# EUROPEAN PATENT APPLICATION

(11) **EP 3 366 368 A1**
(43) Date of publication of application: **29.08.2018**
(21) Application number: 16857532.2
(22) Date of filing: 20.10.2016
(51) Int. Cl.: B01D 65/02, A47K 3/00, A61H 33/02, A61L 2/18, B01D 19/00, B01D 69/08, B01D 69/12, B01F 1/00

(54) **MEMBRANE STERILIZATION METHOD AND GAS-DISSOLUTION LIQUID PRODUCING APPARATUS CAPABLE OF IMPLEMENTING STERILIZATION METHOD**

(30) Priority: 20.10.2015 JP 2015206723
(71) Applicant: Mitsubishi Chemical Corporation, Tokyo 100-8251 (JP)
(72) Inventor: NAKAGAWA, Yoshiichi, Tokyo 141-0032 (JP); KUMAMOTO, Hideo, Tokyo 141-0032 (JP); TERAZAWA, Kaoru, Tokyo 141-0032 (JP); KIKUYA, Nobuyuki, Tokyo 141-0032 (JP); TANAKA, Hiroyuki, Tokyo 141-0032 (JP); ITAKURA, Masanori, Tokyo 141-0032 (JP)
(74) Representative: Hoffmann Eitle
(86) International application number: PCT/JP2016/081178
(87) International publication number: WO 2017/069219

(57) **Abstract**

By contacting a bactericidal agent 2 with a liquid side surface 1b of a membrane 1, contacting a gas 3 with a gas side surface 1a of the membrane, and setting a pressure on the gas side lower than that of the liquid side, wherein a side of one surface 1b of the both surfaces of the membrane 1 is the liquid side and a side of the other surface 1a is the gas side, the inner part of the membrane is highly sterilized. In addition, a pressure difference generating mechanism constituted to perform this sterilization method is imparted to the gas-dissolved liquid manufacturing apparatus.

## Description

### [Technical Field]

The present invention relates to a sterilization method for a membrane, and a gas-dissolved liquid manufacturing apparatus constituted such that the sterilization method can be performed for sterilizing a membrane in the apparatus.

### [Background Art]

Carbonated spring has been used for treatment and bathing for a long time. When the body is immersed in the carbonated spring, the carbon dioxide gas is transdermally absorbed. The vasodilating action thereof has been said to provide an effect of improving diabetes, bedsore, autonomic ataxia, subcutaneous fat increase, peripheral blood circulation failure caused by muscle decrease and the like. In recent years, a superior gas permeable membrane module (in particular, a hollow fiber membrane module) has been developed and is used as a carbonated spring manufacturing apparatus capable of producing and supplying a high concentration carbonated spring on site in various carbonated spring therapies (patent document 1 and the like) .

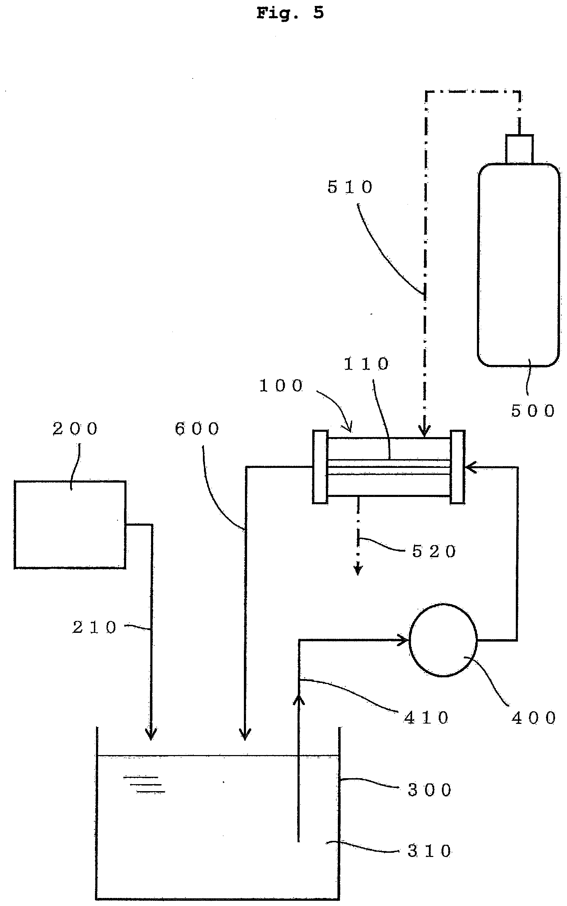

Fig. 5 is a flow diagram schematically showing one embodiment of the constitution of a conventional circulation type carbonated spring producing apparatus. To simplify the illustration, the layout of each element is different from the actual one. In the example shown in the figure, detailed elements such as the open-close valve, pressure reducing device, filter and the like are omitted, and only the relationship between the main flow of water and gas is shown.

As shown in Fig. 5, bathtub 300 contains water 310 supplied from a water (warm water is used) supplying source (water heater etc.) 200 through a supplying flow path 210. The bathtub 300 may be a compact container of a level permitting immersion of a local part such as a foot etc.

In the constituent of a circulation type apparatus, water 310 in the bathtub 300 is suctioned by a pump 400 via a suctioning flow path 410 and fed into a hollow fiber 110 in a hollow fiber membrane module 100. On the other hand, carbon dioxide gas is supplied into the outside of the hollow fiber membrane in the hollow fiber membrane module at an appropriate pressure from a carbon dioxide gas cylinder 500 through a gas supplying flow path 510. In this embodiment, a gas discharging flow path 520 is provided in a hollow fiber membrane module 100. The gas discharging flow path is generally closed by an open-close valve (not shown). In the hollow fiber membrane module, water (inside of the hollow fiber membrane) and carbon dioxide gas (outside of the hollow fiber membrane) are contacted via a hollow fiber membrane, a large amount of carbon dioxide gas permeates through the membrane and is dissolved in water, thus the water turns into high concentration carbonated water (high concentration carbonated spring) while passing through the hollow fiber membrane module. The obtained carbonated water is supplied into a bathtub 300 through a discharge flow path 600.

As mentioned above, since carbonated water has a vasodilating action on transdermal absorption, a carbonated spring therapy is expected to improve peripheral vascular circulatory failure and afford a superior treatment effect on various diseases caused by peripheral vascular circulatory failure such as diabetic foot, decubitus ulcer, ulcer due to burn and the like, and the like.

The point to note here is that the surface layer of the skin (epidermis such as stratum corneum layer, corium and the like) is peeled away, and subcutaneous tissue and the like are exposed (i.e., wound) in diabetic skin ulcer, gangrene, decubitus ulcer and the like. Conventionally, inappropriate warm bath therapy may induce infection in diabetic skin ulcer, and warm bath therapy is considered to be a contraindication. This is because, while keeping a warm bath apparatus until next use after using same, bacteria causing infectious diseases propagate in the water remaining in the warm bath apparatus, may invade the body from the ulcer part at the time of the next warm bath therapy and cause infection. Therefore, also in carbonated spring therapy, when carbonated water is contacted with such wound site and the like, it is necessary to lower the risk of infection by certainly sterilizing the inside of the spring warm bath apparatus after use to prevent infections.

### [Document List]

### [Patent documents]

patent document 1: WO 2001/078883
patent document 2: US Patent No. 7152850, Specification

### [SUMMARY OF THE INVENTION]

### [Problems to be Solved by the Invention]

Sodium hypochlorite is widely used for sterilization and disinfection in a carbonated spring warm bath apparatus. Thus, the present inventors disinfected the inside of a circulation type carbonated spring warm bath apparatus shown in Fig. 5 with sodium hypochlorite, and evaluated the sterilizing effect thereof. As a result, when the inside of a circulation type carbonated spring warm bath apparatus was disinfected with sodium hypochlorite, the inside of the apparatus was appropriately sterilized, and no bacteria were detected during a certain period after the disinfection operation from the carbonated spring produced by the device. Therefore, as long as the carbonated spring warm bath apparatus is used while disinfecting with sodium hypochlorite at an appropriate frequency, bacterial infections is not caused. However, it was found that, when the carbonated spring warm bath apparatus is left for a long term after the disinfection operation, a trace amount of bacteria may be detected from the carbonated spring even if the carbonated spring warm bath apparatus is maintained in a state not open to the outside. This indicates a possibility that bacteria may slightly remain in the carbonated spring warm bath apparatus even though it is disinfected with sodium hypochlorite.

Therefore, the present inventors further studied about the cause thereof and found that the major reason was, as explained below, difficulty in highly sterilizing bacteria that entered a porous membrane in the gas permeable membrane module with a bactericidal agent such as sodium hypochlorite and the like.

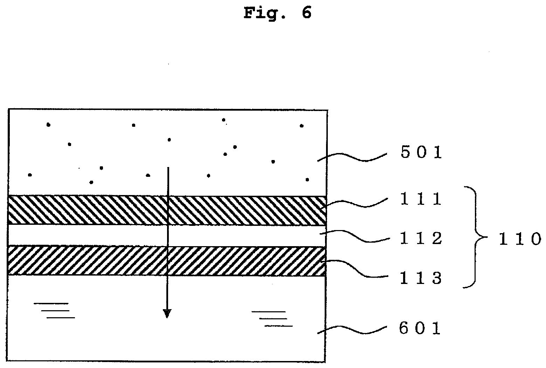

Fig. 6 is a schematic view of a sectional structure of a preferable gas permeable membrane, and is a sectional view of a partially-enlarged wall part of a hollow fiber membrane called a multi-layered composite hollow fiber membrane. The multi-layered composite hollow fiber membrane 110 shown in Fig. 6 has a three-layered structure in which porous layers 111, 113 are respectively laminated on both main surfaces of a non-porous layer 112. A gas 501 is in the outside of the multi-layered composite hollow fiber membrane 110, and water 601 is in a pipe conduit in the inside of the multi-layered composite hollow fiber membrane. In the following explanation, one of the sides of the both main surfaces of the non-porous layer of the gas permeable membrane is referred to as a "gas side" (outside of the multi-layered composite hollow fiber membrane in the embodiment of Fig. 6), and the other side is referred to as a "liquid side" (side of pipe conduit in the inside of the multi-layered composite hollow fiber membrane in the embodiment of Fig. 6).

The non-porous layer is a thin membrane formed from a material having high gas permeability, and does not permeate liquids such as water and the like but permeates only gas components such as oxygen, nitrogen, carbon dioxide gas and the like. The arrow in the Figure suggests a state where a carbon dioxide gas permeates the gas permeable membrane and is dissolved in water. The porous layers 111, 113 are formed from a resin material having high mechanical strength and are supporting layers in support of the non-porous layer 112 from both sides while enabling contact of the gas 501 and water 601 with the non-porous layer by the porous property thereof.

A hydrophobic material is used for the porous layer of the gas permeable membrane as in Fig. 6. The porous layer may be contaminated with bacteria during a membrane production step, a membrane module production step, use and the like. During use of the membrane module (gas permeable membrane module), the liquid side is sterilized using a bactericidal agent. However, the bactericidal agent (aqueous solution of bactericidal agent) does not sufficiently permeate into the inner part of the porous layer made of the hydrophobic material. Therefore, there is a fear that bacteria may remain inside the porous layer, and there is a possibility that the bacteria will proliferate and be released into the carbonated spring. Particularly, when the gas permeable membrane module used once is used again, there is a possibility that a trace amount of bacteria in the porous layer are released into the carbonated water. Such trace amount of bacteria does not pose any problem for general carbonated spring therapy. When carbonated water is applied to a wound site, even the trace amount of bacteria may enter the body through the diseased site. Therefore, to reduce the risk of infection as much as possible, it is desirable to certainly sterilize the bacteria in the porous layer.

The above-mentioned problem of bacteria in the porous layer on the liquid side is a problem that similarly occurs at the second use as in the circulation type when the trace amount of bacteria present in the liquid from the liquid source enters the porous gas permeable membrane, not only in the circulation type apparatus shown in Fig. 5 but also even the constitution of an apparatus called one-way type (constitution without circulation that merely injects the liquid of supply source by passing through the gas permeable membrane module).

Also, the above-mentioned problem of bacteria in the porous membrane is a problem that similarly occurs not only when carbon dioxide gas is dissolved in water but also when other gases such as oxygen, nitrogen, hydrogen and the like are dissolved in a liquid by permeating the gas permeable membrane.

Furthermore, the above-mentioned problem of residual bacteria in the porous membrane is a problem that similarly occurs not only in the gas permeable membrane for dissolving a gas in a liquid but also a gas permeable membrane for separating various gases dissolved in a liquid from the liquid, and further, any porous membrane such as a filtration membrane and the like, through which disinfectants are difficult to infiltrate.

The present invention aims to resolve the above-mentioned problems and provide a sterilization method capable of sterilizing various porous membranes deeper and a gas-dissolved liquid manufacturing apparatus constituted such that the sterilization method can be applied to sterilization of a membrane in the apparatus.

### [Means of Solving the Problems]

The present inventors have conducted intensive studies in an attempt to solve the aforementioned problems and found that the porous membrane to be sterilized can be sterilized deeply thereinto with a liquid bactericidal agent by contacting the liquid bactericidal agent with the liquid side surface of a membrane and reducing the pressure on the gas side relatively lower than that on the liquid side in this state, wherein a side of one of the both surfaces of the membrane is the liquid side and a side of the other surface is the gas side, which resulted in the completion of the present invention.

The main constitution of the present invention is as follows.
[1] A method for sterilizing a membrane, comprising a step of
   contacting a bactericidal agent with a liquid side surface of a membrane, contacting a gas with a gas side surface of the membrane, and setting a pressure on the gas side lower than that on the liquid side, wherein a side of one of the both surfaces of the membrane is the liquid side and a side of the other surface is the gas side.
[2] The sterilization method for a membrane according to [1], wherein
   the gas in contact with the gas side surface of the membrane is soluble in a liquid, an open-close valve formed on a gas flow path leading from the outside to the gas side of the membrane is closed to seal the gas in a state of contact with the gas side surface of the membrane, and the pressure on the gas side is set lower than that on the liquid side by utilizing permeation of the sealed gas through the membrane and dissolution thereof in the bactericidal agent on the liquid side.
[3] The sterilization method for a membrane according to [1] or [2], wherein the gas is a carbon dioxide gas.
[4] The sterilization method for a membrane according to any of [1] - [3], wherein the pressure on the gas side is set lower than that on the liquid side of the membrane by performing either one or both of pressurization of the liquid side of the membrane by a pressurizing device and depressurization of the gas side of the membrane by a pressure reducing device.
[5] The sterilization method for a membrane according to any one of [1] - [4], wherein the membrane is a gas permeable membrane for dissolving a gas in a liquid, or separating a gas from a liquid in which the gas is dissolved.
[6] The sterilization method for a membrane according to [5], wherein the membrane is a gas permeable membrane, and the membrane has a structure in which a porous layer is laminated on the liquid side of a non-porous layer having gas permeability.
[7] The sterilization method for a membrane according to any one of [1] - [5], wherein the membrane is a hollow fiber membrane.
[8] The sterilization method for a membrane according to [7], wherein the membrane is a multi-layered composite hollow fiber membrane and has a three layered structure in which porous layers are laminated with a non-porous layer interposed therebetween.
[9] The sterilization method for a membrane according to any one of [1] - [8], wherein the membrane is a carbon dioxide gas permeable membrane in a membrane module of a carbonated water producing apparatus constituted to produce carbonated water by dissolving a carbon dioxide gas in water.
[10] The sterilization method for a membrane according to [9], wherein the membrane module has a membrane density of 500 - 3000 m²/m³.
[11] A gas-dissolved liquid manufacturing apparatus constituted to produce a gas-dissolved liquid by a membrane module, the apparatus comprising the membrane module for isolating a liquid from a gas by a membrane; and a pressure difference generating mechanism for setting the pressure on a gas side of the membrane lower than the pressure on a liquid side.
[12] The gas-dissolved liquid manufacturing apparatus according to [11], wherein the pressure difference generating mechanism has an open-close valve formed on a gas flow path leading from the outside of the membrane module on the gas side of the membrane, and the gas can be sealed in a state of contact with the membrane by closing the open-close valve.
[13] The gas-dissolved liquid manufacturing apparatus according to [11] or [12], wherein the pressure difference generating mechanism comprises either one or both of a pressurizing device outside the membrane module for pressurizing the liquid side of the membrane and a pressure reducing device outside the membrane module for depressurizing the gas side of the membrane.
[14] The gas-dissolved liquid manufacturing apparatus according to [11] - [13], wherein the membrane has a structure in which a porous layer is laminated on a liquid side of a non-porous layer having gas permeability.
[15] The gas-dissolved liquid manufacturing apparatus according to [14], wherein the membrane is a multi-layered composite hollow fiber membrane and has a three layered structure in which porous layers are laminated with a non-porous layer interposed therebetween.
[16] The gas-dissolved liquid manufacturing apparatus according to [11] - [15], which apparatus being a carbonated water producing apparatus constituted to produce carbonated water by the membrane module, wherein the gas is a carbon dioxide gas and the liquid is water.
[17] The gas-dissolved liquid manufacturing apparatus according to [16], which apparatus being a circulation type carbonated water producing apparatus constituted to suck water contained in the outside tank with a pump, produce carbonated water by the membrane module, and return the carbonated water to the tank to be circulated wherein, when a sterilizing operation of the membrane and the liquid flow path in the apparatus is performed, the bactericidal agent is circulated to be able to contact the liquid side surface of the membrane.
[18] The gas-dissolved liquid manufacturing apparatus according to [16], which apparatus being a one-way type carbonated water manufacturing apparatus constituted to use water supplied from a water supplying source to a supply port, produce carbonated water by the membrane module, and discharge the carbonated water from the outlet port, wherein the supply port and the outlet port are provided with an open-close valve, and the bactericidal agent is retained in a water path between the supply port and the outlet port closed by the open-close valves such that the bactericidal agent is capable of contacting the liquid side surface of the membrane.
[19] The gas-dissolved liquid manufacturing apparatus according to [11] - [18], wherein the membrane module has a membrane density of 500 - 3000 m²/m³.

### [Effect of the Invention]

In the sterilization method of the present invention, in sterilizing the inner part of the membrane, a liquid bactericidal agent is brought into contact with a liquid side surface of the membrane, a gas is brought in contact with a gas side surface of the membrane, and the pressure on the gas side is made lower than the pressure on the liquid side. By this operation, even when the membrane is a water-repellent porous membrane, or when the deepest part of the porous membrane is closed by a non-porous layer, or when the inner part of the porous membrane is already filled with water and the like, a liquid bactericidal agent is considered to enter deep into the membrane due to the pressure difference formed.

In a multi-layered structure composed of a porous layer and a non-porous layer, the gas components in the porous layer move through the non-porous layer to the gas side (lower pressure side) due to pressure difference, and therefore, the inner part of the porous membrane is considered to be depressurized to cause suction and penetration into the porous layer of the bactericidal agent.

In a gas-dissolved liquid manufacturing apparatus represented by a carbonated water manufacturing apparatus, a membrane module for dissolving a gas in a liquid is used. Once a high level sterilization can be achieved for the inner part of the membranes constituting the membrane module, a high level sterilization of other parts of the flow path can be easily achieved merely by flowing a bactericidal agent. Therefore, it becomes possible to safely apply the carbonated water produced by using the gas permeable membrane to the diseased part of the skin.

In the sterilization method of the present invention, moreover, as a method for setting the pressure on the gas side lower than the pressure on the liquid side with a liquid bactericidal agent in contact with a liquid side surface of the membrane is proposed, a method including [contacting a liquid-soluble gas (particularly preferably highly soluble carbon dioxide gas) with the gas side of the membrane, and sealing the aforementioned liquid-soluble gas in a contact state with the membrane by closing all open-close valves on the gas flow path leading to the gas side of the membrane]. According to this method, the sealed liquid-soluble gas (particularly carbon dioxide gas) permeates the membrane and dissolves in the liquid bactericidal agent, and the pressure on the gas side becomes lower than the pressure on the liquid side. Therefore, it is possible to lower the pressure on the gas side and achieve a high level sterilization in the membrane by applying a conventional apparatus configuration.

In addition to the above-mentioned method, the liquid pressure on the liquid side may be raised by an additionally provided pressurizing device and the gas pressure on the gas side may be lowered by an additionally provided pressure reducing device. One or both of the pressurizing and depressurizing by these pressurizing device and pressure reducing device may be performed, or either one or both of them may be used in combination with the above-mentioned method for setting the pressure on a gas side lower than the pressure on a liquid side by dissolving the gas in a liquid bactericidal agent.

In addition, the gas-dissolved liquid manufacturing apparatus of the present invention comprises a pressure difference generating mechanism that enables the sterilization method of the present invention to be implemented. When the pressure difference generating mechanism is operated in a state where a liquid bactericidal agent is in contact with the liquid side surface of the membrane, the pressure on the gas side becomes lower than that on the liquid side, and the inner part of the membrane is highly sterilized.

One of the preferable constitutions of the pressure difference generating mechanism is an open-close valve formed on a gas flow path leading to the gas side of the membrane. By sealing a liquid-soluble gas in a state of contact with the membrane, the liquid-soluble gas is dissolved in a liquid bactericidal agent, and the pressure on the gas side becomes lower than that on the liquid side.

In addition, the pressure difference generating mechanism may be either one or both of a pressurizing device for pressurizing the liquid side of the non-porous layer and a pressure reducing device for depressurizing the gas side of the non-porous layer. The pressure on the gas side can be set lower than that on the liquid side by using these devices, and the liquid side of the gas permeable membrane module can be highly sterilized.

### [Brief Description of the Drawings]

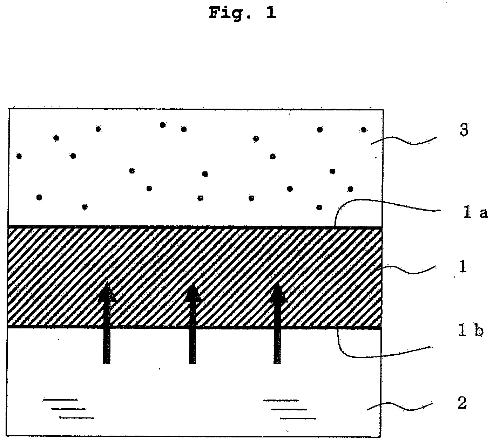

Fig. 1 is a sectional view schematically showing the principle of the sterilization method of the present invention. In this embodiment, the membrane is porous. In the Figure, to distinguish and emphasize the regions, hatching is added to the cross section of the membrane and the presence of gas is indicated by black dots.

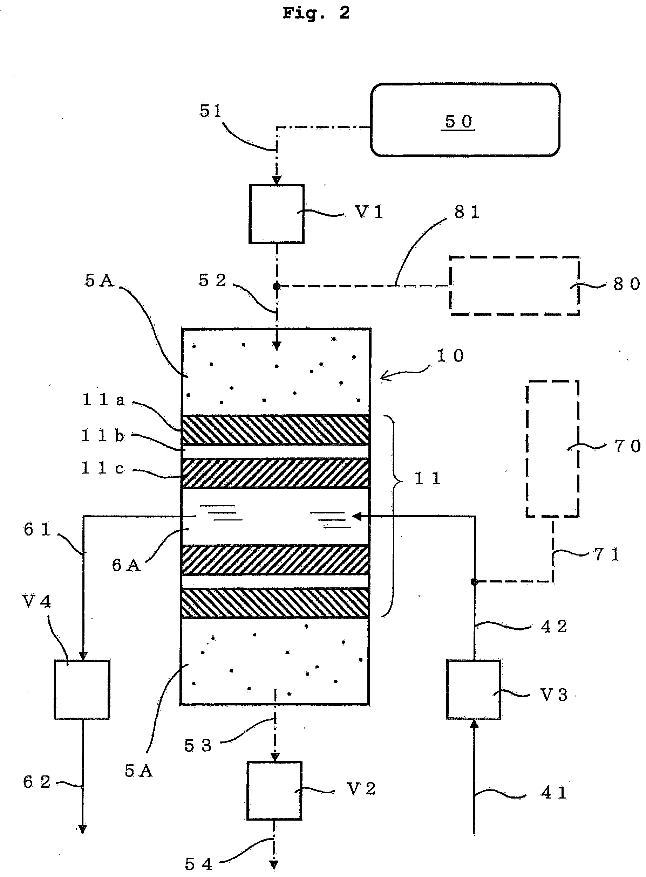

Fig. 2 is a sectional view schematically showing the sterilization method of the present invention and the constitution of an apparatus for implementing the method. In the embodiment in the Figure, the membrane is a gas permeable membrane (in particular, a three-layered composite hollow fiber membrane) in a gas-dissolved liquid manufacturing apparatus, and one composite hollow fiber membrane is enlarged to show the cross section. For explanation, the thickness of the liquid flow path at the center of the composite hollow fiber membrane and the ratio of the thickness of each layer of the composite hollow fiber membrane are different from the actual ones. In this embodiment, to distinguish and emphasize the regions, hatching is added to the cross section of the porous membrane.

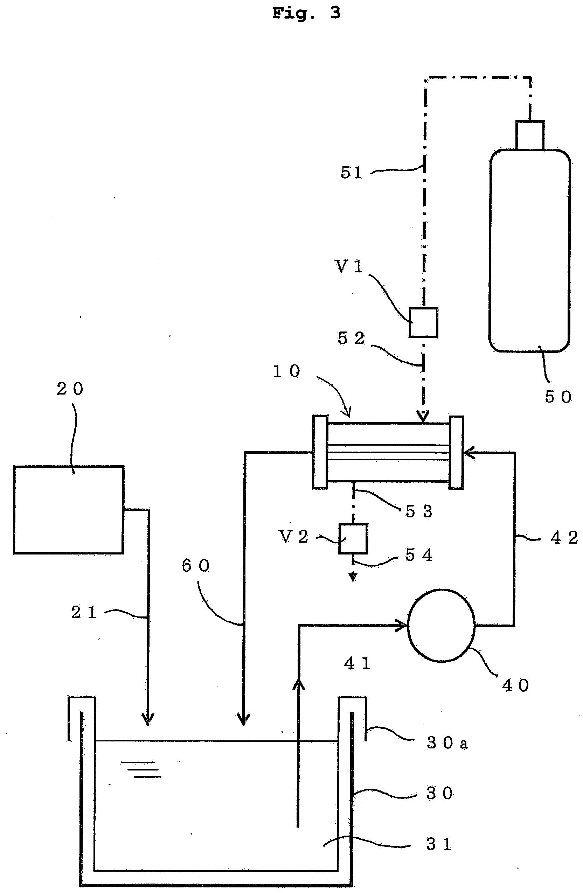

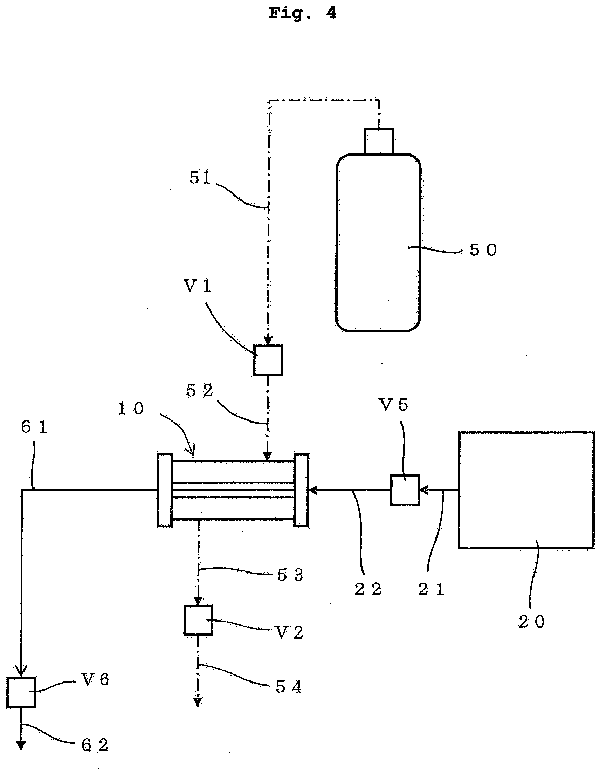

Fig. 3 is a block diagram schematically showing one embodiment of the constitution of the circulation type gas-dissolved liquid manufacturing apparatus of the present invention. In the Figure, except for the open-close valves necessary for carrying out the present invention, all elements in detailed parts such as other open-close valves, pressure reducing devices and the like are omitted, and only the liquid flow path and the gas flow path are shown. The water path is indicated by a solid arrow and the gas flow path is indicated by a dashed line. The direction indicated by each arrow shows the direction of flow (same in Fig. 4).
Fig. 4 is a block diagram schematically showing one embodiment of the constitution of the one-way type gas-dissolved liquid manufacturing apparatus of the present invention.
Fig. 5 is a block diagram schematically showing one embodiment of the constitution of the conventional circulation type carbonated spring producing apparatus. In the Figure, all elements in detailed parts such as open-close valves, pressure reducing devices and the like are omitted, and only the water path and the gas flow path are shown. The liquid flow path (water path) is indicated by a solid arrow and the gas flow path is indicated by a dashed line. The direction indicated by each arrow shows the direction of flow.
Fig. 6 is a schematic view showing the sectional structure of a conventional preferable gas permeable membrane, and is a cross sectional view partially enlarging a wall part of a hollow fiber membrane called a multi-layered composite hollow fiber membrane. Hatching is added for easy visual distinction of the regions.

### [Description of Embodiments]

The present invention is explained in detail in the following by referring to the Figures.

The sterilization method of a membrane of the present invention comprises steps of, as schematically shown in Fig. 1, contacting a liquid bactericidal agent 2 with a liquid side surface 1b of a membrane, contacting a gas 3 with a gas side surface 1a of the membrane, and setting a pressure on the gas side lower than that of the liquid side (liquid bactericidal agent 2), wherein a side of one of the both surfaces (both main surfaces) of the membrane 1 is the liquid side and a side of the other surface 1a is the gas side, whereby, as shown with a thick arrow in the Figure, the liquid bactericidal agent 2 infiltrates into the inner part of the membrane 1.

The membrane for which the sterilization method of the present invention is useful is a membrane having porosity permitting entry of bacteria but having property making entry of a liquid bactericidal agent into the pores difficult. Examples of such membrane include a porous membrane made of a water-repellent material, a gas permeable membrane in which a porous layer on the liquid side (porous membrane layer) and a non-porous layer on the gas side (non-porous membrane layer having gas permeability) are laminated, a filtration membrane and the like.

It is difficult for these membranes to highly sterilize bacteria that entered the membrane by simply running the bactericidal agent through the flow path and bringing the bactericidal agent into contact with the surface of the membrane. Therefore, they are the membranes in which the usefulness of the sterilization method of the present invention becomes prominent.

The sterilization object membrane for which the usefulness of the sterilization method of the present invention is particularly prominent, is a gas permeable membrane used for producing a gas-dissolved liquid such as carbonated water and the like. Among such gas permeable membranes, a bactericidal agent does not easily enter porous layer when the membrane is a gas permeable membrane in which a porous layer on the liquid side (porous membrane layer) and non-porous layer on the gas side (layer of non-porous membrane having gas permeability) are laminated.

Particularly, a carbon dioxide gas permeable membrane for producing carbonated water is sometimes required to show a reduced risk of infection, depending on the application such as carbonated spring therapy and the like. Therefore, the carbon dioxide gas permeable membrane in the carbonated water manufacturing apparatus is a sterilization object for which the sterilization method of the present invention is most useful.

In the following description, a carbon dioxide gas permeable membrane for producing carbonated water is recited as a preferable example of the gas permeable membrane, and a carbonated water manufacturing apparatus having a carbon dioxide gas permeable membrane is recited as a preferable example of the gas-dissolved liquid manufacturing apparatus, and the sterilization method and the gas-dissolved liquid manufacturing apparatus of the present invention are explained.

The explanation of the specific membrane module (gas permeable membrane module) and gas-dissolved liquid manufacturing apparatus described below is also an explanation of how to specifically perform the sterilization method of the present invention. In addition, the explanation of the sterilization method of the present invention is also an explanation of a method for operating the mechanism for sterilizing the gas-dissolved liquid manufacturing apparatus of the present invention.

In the following explanation, water (including warm hot water) in which carbon dioxide is dissolved is widely referred to as "carbonated water" regardless of the temperature of water or the solubility of carbon dioxide. According to the Japanese hot spring law, 1 liter of hot water at not less than 25°C containing not less than 0.25 g (not less than 250 ppm) of carbon dioxide dissolved therein is called "carbonated spring", and when dissolved at not less than 1000 ppm therein, it is recognized as one kind of medical springs. In the present specification, these terms ("carbonated spring", "high concentration carbonated spring") are also used as necessary. When these carbonated spring and high concentration carbonated spring generated artificially are sometimes called "artificial carbonated spring", "high concentration artificial carbonated spring" and the like.

In addition, "water" in the present invention means an aqueous liquid including H₂O, and includes not only purified water containing no impurities but also non-purified water containing impurities such as tap water and the like; an aqueous solution in which a predetermined solute such as sodium chloride, buffering agent and an aqueous solution of a bactericidal agent is dissolved; suspension water (aqueous suspension) in which fine solids capable of passing through the inside of hollow fiber membrane are not dissolved in the water of the base material but dispersed therein; and emulsion water in which fine liquid is dispersed without being dissolved in the water of the base material (aqueous emulsion liquid, aqueous emulsion).

Therefore, the "carbonated water" in the context of the present invention also means a liquid obtained by dissolving carbon dioxide gas in "water". For example, sodium chloride-containing carbonated water obtained by supplying salt water to the carbon dioxide gas permeable membrane module is also "carbonated water". Note that "water" in which a carbon dioxide gas is dissolved is called "carbonated water" irrespective of the temperature of "water" or solubility of carbon dioxide gas, as mentioned above.

The foregoing definition applies to gases other than the carbon dioxide gas.

Fig. 2 schematically shows the sterilization method of the present invention performed in the production of carbonated water, and also schematically shows the sterilizing mechanism of the gas permeable membrane by the gas-dissolved liquid manufacturing apparatus of the present invention (carbonated water manufacturing apparatus in the embodiment of Fig. 2). The embodiment in the Figure is constituted such that the gas permeable membrane 11 is a hollow fiber membrane, and an outside gas 5A is dissolved in a liquid 6A passing through the inside. The wall part of the hollow fiber membrane has a multi-layered structure in which a porous layer 11c is laminated at least on the liquid side of a non-porous layer 11b. In the embodiment in the Figure, a gas permeable membrane 11 is a three-layered composite hollow fiber membrane in which porous layers 11a, 11c are laminated on both sides of the non-porous layer 11b. In this Figure, for explanation, only one composite hollow fiber membrane 11 is enlarged. In fact, however, about 100 - 100000 composite hollow fiber membranes are housed in parallel in the gas permeable membrane module 10 and interposed between the liquid 6A and the gas 5A.

When the sterilization method of the present invention is applied to a gas-dissolved liquid manufacturing apparatus, a liquid bactericidal agent is first contacted with the surface (membrane surface) of the porous layer 11c on the liquid side of the gas permeable membrane 11. In the circulation type apparatus shown in Fig. 3, to contact a bactericidal agent with the surface of the porous layer 11c (Fig. 2) on the liquid side, for example, a bactericidal agent is added to water in a bathtub 30 to give a bactericidal agent solution, and the bactericidal agent solution is circulated along the water path, whereby the bactericidal agent solution can contact the surface of the porous layer 11c. In the case of the one-way type apparatus shown in Fig. 4, for example, bactericidal agent solution is injected into the water path from the water source 20 side, whereby the bactericidal agent solution can contact the surface of the porous layer 11c (Fig. 2) on the liquid side.

In the sterilization method of the present invention, as mentioned above, the pressure on the gas side (pressure of gas 5A in Fig. 2) is set lower than the pressure on the liquid side (pressure of liquid 6A in Fig. 2) in a state where a liquid bactericidal agent is in contact with the surface of the porous layer 11c, whereby the liquid bactericidal agent is impregnated deep into the inside of the porous layer 11c. As a result, the porous layer 11c on the liquid side of the gas permeable membrane (carbon dioxide gas permeable membrane 11 in Fig. 2) is highly sterilized into the inside thereof.

The layer surface (or membrane surface) of the porous layer (or porous membrane) on the liquid side is a surface (surface on the farthest side from gas side) to be in contact with the liquid, assuming the porous layer (or porous membrane) is a non-porous layer (or non-porous membrane).

In the embodiment of Fig. 2, the liquid side surface of the porous layer is a wall surface of the inside flow path of the hollow fiber membrane. When a gas is flown in the inside flow path of the hollow fiber membrane, the outer surface of the body of the hollow fiber membrane is a liquid side surface of the porous layer. Since many pores are open on the surface of the porous layer and irregular concave convex are present, strict definition of the layer surface is difficult and is not important. Even when the surface of the porous layer cannot be strictly classified, there is clearly a problem that the bactericidal agent cannot enter deep inside the porous layer with ease. In the foregoing, therefore, it is stated that a liquid bactericidal agent is brought into contact with the liquid side layer surface of the porous layer, while considering the opening of the pores of the porous layer and the irregular concave convex. When a liquid bactericidal agent is brought into contact with the liquid side layer surface of the porous layer, the bactericidal agent may enter the pores.

In the example where carbon dioxide gas is dissolved in water through a membrane, the time until the liquid bactericidal agent can be impregnated into the inner part of the porous layer on the liquid side (that is, the time required until the pressure on the gas side becomes sufficiently lower than the pressure on the liquid side) varies depending on conditions such as temperature and pressure of water and carbon dioxide, concentration of bactericidal agent and the like. It is generally about 5 seconds - 100 seconds. The state in which the liquid bactericidal agent is impregnated in the inner part of the porous layer on the liquid side is preferably kept for 1 minute or longer as it is for sufficient sterilization.

The aforementioned time is generally the same for the combination of other gas and other liquid.

To clean the liquid bactericidal agent impregnated in the porous layer after sterilizing, for example, rinsing may be performed by passing tap water or the like. Furthermore, when water is passed while applying pressure to the gas side, rinsing can be preferably performed while pushing out the bactericidal agent from the porous layer.

As a method for setting the pressure on the gas side lower than the pressure on the liquid side in a state where the bactericidal agent is in contact with the liquid side layer surface of the porous layer, the present invention proposes a unique depressurizing method.

As shown in Fig. 2, a liquid-soluble gas 5A is contacted with the gas side of the non-porous layer 11b. In the embodiment of Fig. 2, the gas permeable membrane is a three-layered composite hollow fiber membrane, and when the gas 5A is contacted with the gas side layer surface of the porous layer (body outer surface of composite hollow fiber membrane), gas 5A penetrates in the porous layer on the gas side and reaches the gas side surface of the non-porous layer 11b.

Then, the open-close valve on the gas flow path leading to the gas side of the non-porous layer 11b (open-close valve V1 between gas supplying paths 51, 52, open-close valve V2 between gas supplying paths 53, 54 in Fig. 2) is closed, and the liquid-soluble gas 5A is sealed while in the state of contact with the non-porous layer 11b.

By this operation, the sealed liquid-soluble gas permeates the non-porous layer 11b and dissolves in the liquid bactericidal agent. As a result, the pressure on the sealed gas side decreases and becomes lower than that of the pressure on the liquid side.

A method of reducing the pressure on the gas side by dissolving the liquid-soluble gas in the liquid bactericidal agent by passing through the non-porous layer as described above is also referred to as a "gas-dissolving and depressurizing method" in the following explanation.

In the sterilization step, the pressure on the gas side may be lower than the pressure on the liquid side. When the pressure difference between them is not less than 5kPa, the degree of penetration of the bactericidal agent becomes more pronounced, and when the pressure difference between them is 20kPa to 500kPa, the degree of penetration of the bactericidal agent becomes particularly notable, which is preferable. When the pressure difference exceeds the above range and becomes excessively large, the gas permeable membrane suffers damage such as breakage, which is not preferable.

The liquid-soluble gas usable for the gas-dissolving and depressurizing method may be any as long as it can be dissolved in a liquid, and examples thereof include carbon dioxide gas, oxygen, nitrogen, hydrogen, and the like. Among them, carbon dioxide gas is a gas having significantly higher solubility than other gases such as oxygen, nitrogen, hydrogen and the like, and is particularly preferable for lowering the pressure on the gas side.

When the gas-dissolved liquid manufacturing apparatus is a carbonated water manufacturing apparatus, since a carbon dioxide gas supply source is attached to the apparatus, carbon dioxide gas can be used as a liquid-soluble gas in the sterilization step without addition of any special gas supply device. Thus, the usefulness of the present invention becomes particularly remarkable.

In the case of a carbonated water manufacturing apparatus using a gas permeable membrane, the pressure (absolute pressure) of carbon dioxide gas in the gas permeable membrane module is set to about 0.1 MPa to 0.5 MPa by a pressure reducing valve. On the other hand, the pressure on the water side at that time is equal to the atmospheric pressure in the water path of a general carbonated water manufacturing apparatus. To promote dissolution of carbon dioxide gas in water, the pressure of carbon dioxide gas in the gas permeable membrane module is set to be higher than the pressure on the water side.

To preferably carry out the gas-dissolving and depressurizing method in the sterilizing step from such use state, the gas side pressure of the gas permeable membrane module sealed in contact with the membrane is preferably 0.10 MPa to 0.13 MPa, and approximately atmospheric pressure is more preferable. In the gas-dissolving and depressurizing method, to set the pressure of the carbon dioxide gas to be sealed to a level lower than the atmospheric pressure, another means is required. When the pressure is higher than 0.13MPa, depressurization due to dissolution becomes insufficient and there arises a problem that penetration of the bactericidal agent into the porous layer on the liquid side becomes insufficient.

In the above-mentioned sterilizing step, as other method of reducing the pressure on the gas side lower than that on the liquid side, the liquid side may be mechanically pressurized or the gas side may be mechanically depressurized. Either one or both of the mechanical pressurization on the liquid side and the mechanical depressurization on the gas side may be performed.

Either one or both of the mechanical pressurization on the liquid side and the mechanical depressurization on the gas side may be performed concurrently with the above-mentioned gas-dissolving and depressurizing method.

As regards the mechanism for the pressurization and depressurization, a more specific configuration will be shown in the explanation of the apparatus to be described later.

The gas permeable membrane may be at least a porous membrane, and more preferably has a multi-layered structure in which a porous layer is laminated on the liquid side of the non-porous layer, more preferably a hollow fiber membrane, particularly preferably a multi-layered composite hollow fiber membrane.

The composite hollow fiber membrane having a three-layered structure described in the above-mentioned patent document 1 is particularly preferable since it is less likely to damage the non-porous membrane at the time of membrane production, module production and use.

When a gas is dissolved in a liquid by using a hollow fiber membrane, the liquid may be flown to the outside of the hollow fiber membrane and the gas may be flown inside the hollow fiber membrane. For uniform contact of the liquid with the membrane surface, it is preferable to flow the liquid inside the hollow fiber membrane and flow the gas to the outside of the hollow fiber membrane.

In the membrane to be sterilized, the larger one of the total area of the membrane surface on the liquid side and the total area of the membrane surface on the gas side is referred to as "membrane area of the membrane module". As used herein, the "total area of the membrane surface" means the total area of the surface of all membranes in the membrane module. The total area of the membrane surface on the liquid side can be calculated by multiplying the area of the surface of one membrane (when membrane is hollow fiber membrane, one hollow fiber membrane) on the liquid side by the total number of membranes in the membrane module. The total area of the membrane surface on the gas side can be calculated by multiplying the area of the surface of one membrane (when membrane is hollow fiber membrane, one hollow fiber membrane) on the gas side by the total number of membranes in the membrane module.

In the case of a composite hollow fiber membrane having a three-layered structure in which porous layers are disposed on both surfaces of a non-porous layer, the membrane area of the membrane module refers to the larger of the total areas (that is, the total area of the surface on the internal flow path side of the composite hollow fiber membrane and the total area of the surface on the outside of the body of the composite hollow fiber membrane) of the layer surfaces on the outside of each of the two porous layers. The "total area of the layer surface" is synonymous with the "total area of the membrane surface" and can be calculated similarly.

The volume of the space in which the membrane except for the connection part for suctioning or passing the liquid is arranged (the sum of the volume of the membrane and the volume of the interior space surrounded by the membrane) is referred to as "membrane module volume". In the case of a hollow fiber membrane, the space in which the hollow fiber membrane is disposed refers to the sum of the volume of the membrane part of the hollow fiber membrane and the volume of the internal flow path of the hollow fiber membrane.

The value obtained by dividing the "membrane area of the membrane module" by the "membrane module volume" is referred to as "membrane density of membrane module".

The membrane density of the membrane module is preferably 500 - 3000 m²/m³, more preferably 1000 - 1500 m²/m³, since the total number of the membranes does not become too many, the size of the membrane module does not become too large, and the gas flow path is sufficiently secured to efficiently dissolve the gas.

A preferable material of the non-porous layer includes polyethylene, polypropylene, polyurethane and the like.

A preferable thickness of the non-porous layer is about 0.1 µm - 20 µm.

A preferable material of the porous layer on the liquid side includes polyethylene, polypropylene, polysulphone, polyethersulfone, polyvinyl chloride and the like.

Generally, a hydrophobic material is used as the gas permeable membrane because it is necessary to dissolve the gas without allowing the liquid to pass through. Even when a non-porous layer is present inside, a hydrophobic material is similarly used since a porous layer made from hydrophilic material may cause an obstacle to gas dissolution. A preferable thickness of the porous layer is about 10 µm - 200 µm.

The specification of the aforementioned porous layer may be the same as that of the porous layer on the gas side.

Examples of the bacteria to be the target of sterilization include, but are not limited to, pathogenic bacteria such as legionella bacteria, pseudomonas aeruginosa, staphylococcus aureus and the like, virus and the like.

The bactericidal agent usable in the present invention may be a solid and may be any as long as it can be dissolved in a liquid and can be brought into contact with the surface of the membrane as a liquid at the time of use.

Examples of the bactericidal agent include, but are not limited to, hypochlorite (e.g., sodium hypochlorite, calcium hypochlorite), chlorinated isocyanuric acid, chlorine dioxide, silver ion, hydrogen peroxide and the like. From the viewpoint of uniform dissolution in liquid, safety and broad utility, the bactericidal agent is preferably hypochlorite, more preferably sodium hypochlorite.

The concentration of the bactericidal agent may be appropriately determined depending on the bacterium to be the target and the intended use. For example, in the case of hypochlorite, about 0.1 ppm - 200 ppm is preferable.

Among the above-mentioned bactericidal agents, hypochlorite shows a strong sterilizing effect in the weak acidic range.

The property of the hypochlorite shows a special sterilizing action when the sterilization method of the present invention is applied to the sterilization of the porous layer on the liquid side of the carbon dioxide gas permeable membrane in the carbonated water manufacturing apparatus and the aforementioned gas-dissolving and depressurizing method is applied. This is because, when performing the gas-dissolving and depressurizing method in the carbonated water manufacturing apparatus, carbon dioxide gas is dissolved in the sodium hypochlorite solution that has entered the porous layer on the liquid side, the sodium hypochlorite solution becomes weakly acidic in the porous layer on the liquid side and exerts strong sterilizing activity.

The level of sterilization of the inside of the porous layer on the liquid side of the gas permeable membrane can be determined, for example, by standing unsterilized tap water sealed in a gas permeable membrane module in an incubator at 25°C for a given time, culturing the sample water (1 ml) in the module in a standard agar medium for about 2 days, and measuring the colony.

The sterilizing state achievable by the present invention is a state in which, for example, when unsterilized tap water is sealed in a gas permeable membrane module containing a porous membrane sterilized by the present invention and proliferation of bacteria if not found in the aforementioned sealed tap water even after lapse of a period of not less than one month.

Next, the configuration of the gas-dissolved liquid manufacturing apparatus of the present invention is described. In the following explanation, a carbonated water manufacturing apparatus is cited as an example of a gas-dissolved liquid manufacturing apparatus. The constitution for sterilizing a membrane is the same as that of a gas-dissolved liquid manufacturing apparatus for dissolving a gas other than carbon dioxide gas in a liquid.

The gas-dissolved liquid manufacturing apparatus of the present invention is constituted to produce a gas-dissolved liquid (carbonated water) by the gas permeable membrane module 10 as an example of a carbonated water manufacturing apparatus shown in Fig. 2. The configuration itself for dissolving the gas in the liquid through the membrane is the same as that of the conventionally-known apparatuses.

The gas permeable membrane in the gas permeable membrane module 10 has a multi-layered structure in which a porous layer 11c is laminated on at least the liquid side of the gas side and the gas side of the non-porous layer 11b.

The constitution of each part of the apparatus of Fig. 2, the detail of the gas permeable membrane, the conditions during sterilization and the like are as described in the explanation of the sterilization method of the present invention.

The gas-dissolved liquid manufacturing apparatus has a pressure difference generating mechanism so that the porous layer 11c on the liquid side can be highly sterilized. When the water path including the liquid side of the gas permeable membrane is sterilized by a liquid bactericidal agent, that is, in a state where the liquid bactericidal agent is in contact with the layer surface of the porous layer on the liquid side, the pressure difference generating mechanism operates by manual operation or by mechanical power driving. By the operation of the pressure difference generating mechanism, the pressure on the gas side is lower than the pressure on the liquid side of the nonporous layer, the liquid bactericidal agent in contact with the layer surface of the aforementioned porous layer enters the porous layer, and the inner part of the porous layer is highly sterilized.

The pressure difference generating mechanism may be a mechanism having one or more of the configuration for carrying out the gas-dissolving and depressurizing method described in the explanation of the sterilization method of the present invention, the configuration in which the liquid side of the non-porous layer 11b is mechanically pressurized, and the configuration in which the gas side of the non-porous layer 11b is mechanically depressurized.

Fig. 2 also shows a constitution example of the pressure difference generating mechanism as a block diagram.

The mechanism using open-close valve V1 and open-close valve V2 for carrying out the gas-dissolving and depressurizing method is as described in the explanation of the sterilization method of the present invention. In the following, examples of a mechanism configured to mechanically pressurize the liquid side of the non-porous layer 11b and examples of a mechanism configured to mechanically depressurize the gas side of the nonporous layer 11b are explained.

The flow path of a liquid includes, in the order from the supply side of liquid (pump side in the circulation type of Fig. 3, and water source side in the one-way type of Fig. 4), supply flow path 41, open-close valve V3, supply flow path 42, gas permeable membrane module 10, discharge flow path 61, open-close valve V4, and discharge flow path 62.

A liquid supply flow path 42 branches into a pressurizing flow path 71, and the pressurizing flow path 71 is connected to a pressurizing device 70. The pressurizing device 70 is configured so as to be able to supply liquid to the supply flow path 42 in a state where the open-close valves V3, V4 are closed, thereby pressurizing the liquid side of the non-porous layer 11b.

On the other hand, a gas supplying flow path 52 branches into a depressurizing flow path 81 and the depressurizing flow path 81 is connected to a pressure reducing device 80. The pressure reducing device 80 is configured so as to be able to suck gas from the supply flow path 42 in a state where the open-close valves V1, V2 are closed, thereby depressurizing the gas side of the non-porous layer 11b.

The pressurizing device 70, pressure reducing device 80 may be operated manually. As a pressurizing device operated by a signal from the control unit, a syringe (used as a discharging device), a pump, a compressor and the like are preferable. As a pressure reducing device operated by a signal from the controller, a syringe (used as suctioning device), an aspirator, a vacuum pump and the like are preferable.

To implement the gas-dissolving and depressurizing method or mechanical pressurization of the non-porous layer on the liquid side and mechanical depressurization of the non-porous layer on the gas side, open-close valves V1, V2, V3, V4 may be each operated manually or operated by a mechanical power source such as solenoid valve operated by a signal from the controller (not shown) and the like.

Preferably, the timing of operation of each of the open-close valves V1, V2, V3, V4, pressurizing device 70 and pressure reducing device 80 is controlled by a controller. For example, when the liquid side of the non-porous layer is pressurized, the pressurizing device 70 is operated by an actuating signal from the controller after open-close valves V3, V4 are closed by an actuating signal from the controller.

A sensor for detecting a bactericidal agent may be provided on the liquid side of the membrane (for example, inner wall of outlet port of gas permeable membrane module) and contact of the bactericidal agent with the layer surface of the porous layer on the liquid side in the gas permeable membrane may be informed to the user by turning on of an indicator lamp, display on the monitor screen of the controller, or the like.

In addition, the controller may be configured to automatically close the open-close valves V1, V2 and automatically perform the gas-dissolving and depressurizing method when the sensor detects a bactericidal agent. At that time, the controller may also actuate the pressure reducing device 80 to promote reduction of the pressure on the gas side.

Similarly, the controller may automatically close the open-close valves V3, V4 and actuate the pressurizing device 70 to promote increase of the pressure on the liquid side when the sensor detects a bactericidal agent.

A pressure sensor may be provided on the gas side and the liquid side of the non-porous layer so as to monitor the effect of the operation of the pressure difference generating mechanism. Also, the controller may be configured to automatically control the pressure difference generating mechanism and adjust the pressure difference based on the results of the monitoring.

The controller may be a combination of a computer and a driver of each solenoid valve, or may be a relay circuit configured to be able to perform sequential control or the like.

Fig. 3 is a flow diagram schematically showing one embodiment of the constitution of the circulation type gas-dissolved liquid manufacturing apparatus (carbonated water manufacturing apparatus) of the present invention. To simplify the illustration, the configuration of each element is different from the actual one. The open-close valves V1, V2 are for depressurizing the gas side, and only the relationship between the main flow of water and gas is shown by omitting all the details of other valves, pressure reducing device, filter and the like.

In the embodiment of Fig. 3, a bathtub is an accessory part of the carbonated water manufacturing apparatus. The bathtub has a dual structure, and a thin sac-like inner tank made from polyethylene, polypropylene, polyethylene terephthalate, polyester, polyvinyl chloride, nylon or the like is set in the inside of the outer tank 30. The inner tank is disposable, thereby reducing the risk of infection due to the intervention of the bathtub.

The constitution itself of the circulation type water path, gas flow path is the same as that of the conventional example of Fig. 5, and water 31 supplied from the source of water (warm water) (water heater etc.) 20 through a supply flow path 21 is contained in a bathtub. Water may be supplied through a filter (not shown). A bathtub 30 may be a general bathtub in which the whole body can be immersed, or a compact container in which a topical part such as foot and the like can be immersed. It may be a bathtub in which an animal can bathe. Water 31 in a bathtub is sucked by a pump 40 through a supply flow path 41, passes through a water path 42 and delivered into a hollow fiber membrane in a hollow fiber membrane module 10. On the other hand, a carbon dioxide gas is supplied to the outside of the hollow fiber membrane in the hollow fiber membrane module at an appropriate pressure from a carbon dioxide gas cylinder 50 through gas supplying flow paths 51, 52. In the embodiment in the Figure, the hollow fiber membrane module 10 is provided with gas discharging flow paths 53, 54. The gas discharging flow path is generally closed by the open-close valve V2. In the hollow fiber membrane module, water (in hollow fiber membrane) contacts a carbon dioxide gas (outside of hollow fiber membrane) via a hollow fiber membrane, a large amount of carbon dioxide gas permeates the membrane and dissolves in water, and the water turns into high concentration carbonated water (high concentration carbonated spring) during passage through the hollow fiber membrane module. The obtained carbonated water is supplied to a bathtub through a discharging flow path 60. In this manner, the produced carbonated water can circulate via a bathtub.

During sterilization, for example, a bactericidal agent is added into the bathtub, water containing the bactericidal agent (i.e., liquid bactericidal agent) is circulated to bring the liquid bactericidal agent in contact with a liquid side layer surface of the porous layer of the gas permeable membrane.

In the apparatus of Fig. 3, an open-close valve V1 is inserted between gas supplying flow paths 51 and 52, and the gas-dissolving and depressurizing method can be performed during sterilization. An open-close valve V2 is normally closed. When the open-close valve V1 is closed during supply of carbon dioxide gas, the gas-dissolving and depressurizing method is performed.

A pressure reducing device for depressurizing the gas side of a non-porous layer and a pressurizing device for pressurizing the liquid side of a non-porous layer may be added as appropriate according to the constitution of Fig. 2.

Fig. 4 is a flow diagram schematically showing one embodiment of the constitution of the one-way type gas-dissolved liquid manufacturing apparatus (carbonated water manufacturing apparatus) of the present invention. To simplify the illustration, the configuration of each element is different from the actual one. As in Fig. 3, the open-close valves V1, V2 are for depressurizing the gas side, and only the relationship between the main flow of water and gas is shown by omitting all the details of other valves, pressure reducing device, filter and the like.

In the constitution of a one-way type flow path, water (warm water) is supplied from the source of supply (water heater etc.) 20 through a supply flow path 21, 21 and fed into the hollow fiber membrane of the gas permeable membrane module 10 to provide high concentration carbonated water and the water is discharged to the outside through discharge flow paths 61, 62. The gas supplying flow paths 51, 52 from carbon dioxide gas cylinder 50, gas discharging flow paths 53, 54 from gas permeable membrane module 10 and open-close valves V1, V2 are the same as those in the circulation type shown in Fig. 3.

An open-close valve V5 is provided at the inlet of the entire water path and an open-close valve V6 is provided at the outlet of the entire water path so that the bactericidal agent can be effectively brought into contact with the entire water path at the time of sterilization. The supply flow path 21 in the embodiment of Fig. 4 may be a pipe conduit on the source side or a short pipe conduit attached to the open-close valve V5. The discharge flow path 62 in the embodiment of Fig. 4 may be a detachable short nozzle for discharge, or a short pipe conduit attached to the open-close valve V6. The water path between the open-close valve V5 and open-close valve V6 is preferably as short as possible to reduce the volume of holding water (minimize possibility of bacterial propagation).

In addition to the above-mentioned open-close valves V5, V6, as shown in Fig. 2, open-close valves V3, V4 may be separately provided as a pressure difference generating mechanism in the vicinity of the permeation membrane module 10.

During sterilization, a liquid bactericidal agent may be continuously flown in the water path. However, if a liquid bactericidal agent is filled in a water path between the inlet closed by the open-close valves V5, V6 and the outlet and this state is maintained, a predetermined amount of the liquid bactericidal agent can be effectively and economically brought into contact with the liquid side layer surface of the porous layer in the gas permeable membrane.

The above-mentioned circulation type apparatus may be constituted to be usable as a one-way type by switching the flow path. For example, in Fig. 3, when the water path from the water supplying source 20 can be switched to a water path that directly enters the permeable membrane module 10 without going through the bathtub, the apparatus can also be used as a one-way type.

The gas-dissolved liquid manufacturing apparatus of the present invention is characterized in that the liquid side of the gas permeable membrane can be highly sterilized. The use of the gas-dissolved liquid manufacturing apparatus is not particularly limited, and a carbonated water manufacturing apparatus enables use of carbonated water (carbonated spring and high concentration carbonated spring) for the treatment of, for example, diabetic foot, decubitus ulcer, skin ulcer due to burn and the like, gangrene and the like. In particular, in the treatment of wounds caused by peripheral blood circulation failure such as diabetic foot lesions with carbonated water, the sterilizing ability of the apparatus of the present invention is remarkably exhibited and is particularly useful. For example, by applying a therapeutically effective amount (i.e., peripheral vasodilating dose) of the carbonated water produced by the carbonated water producing apparatus of the present invention to the peripheral blood circulation insufficiency site (preferably, wound site caused by peripheral blood circulation failure) in patients with peripheral blood circulation failure, peripheral blood circulation failure in the patients and wounds resulting therefrom are treated. Application of carbonated water to a peripheral blood circulation insufficiency site (preferably, wound site caused by peripheral blood circulation failure) can be conducted by immersing the site in the carbonated water, or washing the site with the carbonated water. After completion of the application of the carbonated water, the gas permeable membrane in the carbonated water manufacturing apparatus used is sterilized by the sterilization method of the present invention, and the carbonated water manufacturing apparatus is stored under an appropriate hygiene environment until the next use. When in use the next time, the carbonated water produced by the carbonated water manufacturing apparatus of the present invention in which the gas permeable membrane is sterilized by the sterilization method of the present invention is applied to a peripheral blood circulation insufficiency site (preferably, wound site caused by peripheral blood circulation failure) of a patient with peripheral blood circulation failure (may be the same as or different from the patient in previous use) to treat the peripheral blood circulation failure of the patient or a wound caused thereby. In this way, by alternately repeating the treatment operation and the sterilization operation by using the apparatus of the present invention, a carbonated spring therapy of peripheral blood circulation failure and diseases caused thereby (wound etc.) can be continuously carried out while suppressing the risk of the onset of infectious diseases as much as possible.

### [Examples]

As Example of the sterilization method and gas-dissolved liquid manufacturing apparatus of the present invention, a gas permeable membrane module for carbonated water manufacturing apparatus was used and the sterilization method of the present invention was performed on the gas permeable membrane module to confirm the sterilizing effect thereof.

The gas permeable membrane module used was a gas permeable membrane module MHF0504 manufactured by Mitsubishi Rayon, containing 7800 composite hollow fiber membranes with three-layered structures.

The specification of each part of the composite hollow fiber membrane is as follows:
porous layer on the gas side (outside of hollow fiber membrane); material polyethylene, layer thickness about 40 µm,
porous layer on the liquid side (inside of hollow fiber membrane); material polyethylene, layer thickness about 40 µm,
inner non-porous layer; material polyurethane, layer thickness about 1 µm.

### (Example 1)

A carbon dioxide gas was supplied to the gas side of the above-mentioned gas permeable membrane module by the following steps (a) - (f), unsterilized tap water (containing legal amount of sodium hypochlorite in Japan) was supplied to the liquid side (water path), the porous layer of the gas permeable membrane module was impregnated with the tap water to perform sterilization, and the proliferation of bacteria was observed.
(a) A water supplying pump, a piping tube and a carbonic acid cylinder are connected to the gas permeable membrane module, and unsterilized tap water is supplied to the liquid side (water path) for 5 min while supplying carbon dioxide gas to the gas side of the gas permeable membrane module.
(b) Supply of the carbon dioxide gas to the module is ceased, the gas supply side is sealed, and tap water (unsterilized) is passed through for 5 min. By this operation, carbon dioxide gas dissolves in tap water and the gas-dissolving and depressurizing method is performed.
(c) The gas pressure on the gas side (outside of hollow fiber membrane) of the gas permeable membrane module is measured, and negative pressure (about -30kPa) is confirmed (confirmation that gas-dissolving and depressurizing method is performed).
(d) A gas supply flow path, a water supply/discharge flow path are sealed and set in an incubator at 25°C.
(e) Sample water in the gas permeable membrane module is collected on day 0 (initial), day 2, day 4, day 6, day 8, day 10, day 14, day 21, day 28 and day 35.
(f) The collected sample (1 ml) is added to a standard agar plate, and the number of bacteria in the collected water is measured for each elapsed time from the number of colonies developed on the plate by 3 days later.

### (Results)

Bacterial count was zero for all collection dates (n = 3).

Therefrom it was found that tap water sufficiently penetrated into the porous layer on the water path side (inside of the hollow fiber membrane), bacterial proliferation in the porous layer was suppressed by the sterilizing property of sodium hypochlorite contained in tap water.

### (Comparative Example 1)

In the above-mentioned Example 1 (a), carbon dioxide gas is not supplied, unsterilized tap water is supplied for 5 min to the liquid side (water path), discontinuation of carbon dioxide gas supply and sealing of gas supply are not performed in (b), and tap water (unsterilized) is passed for 5 min. (c) is not performed, and sample water is collected from gas permeable membrane module on day 0 (initial), day 2, day 4, day 6 and day 8 in (e) .

The treatment was performed in the same manner as in the above-mentioned Example 1 except for these changes, and the results shown in the following Table 1 were obtained.

**Table 1**

| | initial | day 2 | day 4 | day 6 | day 8 |
|---|---|---|---|---|---|
| No. 1 | 0 | 0 | 0 | 0 | 730 |
| No. 2 | 0 | 1 | 0 | 4,100 | 36,000 |
| No. 3 | 0 | 0 | 0 | 1,200 | 51,000 |

From the results of the above-mentioned Table 1, it was found that bacteria proliferated in the porous layer when tap water was not penetrated into the porous layer on the water path side (hollow fiber membrane inside side) by not setting the gas side under negative pressure.

### (Comparative Example 2)

In the above-mentioned Example 1 (a), carbon dioxide gas is not supplied, 200 ppm aqueous solution of hypochlorous acid is supplied for 5 min to the liquid side (water path), discontinuation of carbon dioxide gas supply and sealing of gas supply are not performed in (b), and tap water (unsterilized) is passed for 5 min. (c) is not performed, and sample water is collected from gas permeable membrane module on day 0 (initial), day 2, day 4, day 6 and day 8 in (e).

The treatment was performed in the same manner as in the above-mentioned Example 1 except for these changes, and the results shown in the following Table 2 were obtained.

**Table 2**

| | initial | day 2 | day 4 | day 6 | day 8 |
|---|---|---|---|---|---|
| No. 1 | 0 | 230 | 700,000 | 1,100,000 | 1,100,000 |
| No. 2 | 0 | 0 | 0 | 1 | 140 |
| No. 3 | 0 | 0 | 3,000 | 1,400 | 110,000 |

From the results of the above-mentioned Table 2, it was found that, when the gas side is not set to negative pressure, the aqueous hypochlorous acid solution did not enter into the porous layer, and bacteria proliferated in the porous layer even when 200 ppm aqueous hypochlorous acid solution having higher sterilizing power is used in the porous layer on the water path side (inside of hollow fiber membrane).

### [Industrial Applicability]

According to the sterilization method of the present invention, the inner part of a membrane into which a liquid cannot enter easily (particularly, a porous layer on the liquid side of a gas permeable membrane module in a carbonated water manufacturing apparatus) can be highly sterilized. In addition, the gas-dissolved liquid manufacturing apparatus of the present invention can maintain the inner part of the membrane in the gas permeable membrane module in a highly sterilized state. In the case of, for example, a carbonated water manufacturing apparatus, a carbonated spring therapy of peripheral blood circulation failure and diseases caused thereby (wound etc.) can be continuously carried out while suppressing the risk of the onset of infectious diseases as much as possible.

This application is based on a patent application No. 2015-206723 filed in Japan (filing date: October 20, 2015), the contents of which are incorporated in full herein.

### [Explanation of Symbols]

1 membrane
1a surface on the gas side
1b surface on the liquid side
2 bactericidal agent (liquid)
3 gas

## Claims

1. A method for sterilizing a membrane, comprising a step of
contacting a bactericidal agent with a liquid side surface of a membrane, contacting a gas with a gas side surface of the membrane, and setting a pressure on the gas side lower than that on the liquid side, wherein a side of one of the both surfaces of the membrane is the liquid side and a side of the other surface is the gas side.

2. The sterilization method for a membrane according to claim 1, wherein
the gas in contact with the gas side surface of the membrane is soluble in a liquid, an open-close valve formed on a gas flow path leading from the outside to the gas side of the membrane is closed to seal the gas in a state of contact with the gas side surface of the membrane, and the pressure on the gas side is set lower than that on the liquid side by utilizing permeation of the sealed gas through the membrane and dissolution thereof in the bactericidal agent on the liquid side.

3. The sterilization method for a membrane according to claim 1 or 2, wherein the gas is a carbon dioxide gas.

4. The sterilization method for a membrane according to any one of claims 1 to 3, wherein the pressure on the gas side is set lower than that on the liquid side of the membrane by performing either one or both of pressurization of the liquid side of the membrane by a pressurizing device and depressurization of the gas side of the membrane by a pressure reducing device.

5. The sterilization method for a membrane according to any one of claims 1 to 4, wherein the membrane is a gas permeable membrane for dissolving a gas in a liquid, or separating a gas from a liquid in which the gas is dissolved.

6. The sterilization method for a membrane according to claim 5, wherein the membrane is a gas permeable membrane, and the membrane has a structure in which a porous layer is laminated on the liquid side of a non-porous layer having gas permeability.

7. The sterilization method for a membrane according to any one of claims 1 to 5, wherein the membrane is a hollow fiber membrane.

8. The sterilization method for a membrane according to claim 7, wherein the membrane is a multi-layered composite hollow fiber membrane and has a three layered structure in which porous layers are laminated with a non-porous layer interposed therebetween.

9. The sterilization method for a membrane according to any one of claims 1 to 8, wherein the membrane is a carbon dioxide gas permeable membrane in a membrane module of a carbonated water producing apparatus constituted to produce carbonated water by dissolving a carbon dioxide gas in water.

10. The sterilization method for a membrane according to claim 9, wherein the membrane module has a membrane density of 500 - 3000 m²/m³.

11. A gas-dissolved liquid manufacturing apparatus constituted to produce a gas-dissolved liquid by a membrane module, the apparatus comprising the membrane module for isolating a liquid from a gas by a membrane; and a pressure difference generating mechanism for setting the pressure on a gas side of the membrane lower than the pressure on a liquid side.

12. The gas-dissolved liquid manufacturing apparatus according to claim 11, wherein the pressure difference generating mechanism has an open-close valve formed on a gas flow path leading from the outside of the membrane module on the gas side of the membrane, and the gas can be sealed in a state of contact with the membrane by closing the open-close valve.

13. The gas-dissolved liquid manufacturing apparatus according to claim 11 or 12, wherein the pressure difference generating mechanism comprises either one or both of a pressurizing device outside the membrane module for pressurizing the liquid side of the membrane and a pressure reducing device outside the membrane module for depressurizing the gas side of the membrane.

14. The gas-dissolved liquid manufacturing apparatus according to any one of claims 11 to 13, wherein the membrane has a structure in which a porous layer is laminated on a liquid side of a non-porous layer having gas permeability.

15. The gas-dissolved liquid manufacturing apparatus according to claim 14, wherein the membrane is a multi-layered composite hollow fiber membrane and has a three layered structure in which porous layers are laminated with a non-porous layer interposed therebetween.

16. The gas-dissolved liquid manufacturing apparatus according to any one of claims 11 to 15, which apparatus being a carbonated water producing apparatus constituted to produce carbonated water by the membrane module, wherein the gas is a carbon dioxide gas and the liquid is water.

17. The gas-dissolved liquid manufacturing apparatus according to claim 16, which apparatus being a circulation type carbonated water producing apparatus constituted to suck water contained in the outside tank with a pump, produce carbonated water by the membrane module, and return the carbonated water to the tank to be circulated wherein, when a sterilizing operation of the membrane and the liquid flow path in the apparatus is performed, the bactericidal agent is circulated to be able to contact the liquid side surface of the membrane.

18. The gas-dissolved liquid manufacturing apparatus according to claim 16, which apparatus being a one-way type carbonated water manufacturing apparatus constituted to use water supplied from a water supplying source to a supply port, produce carbonated water by the membrane module, and discharge the carbonated water from the outlet port, wherein the supply port and the outlet port are provided with an open-close valve, and the bactericidal agent is retained in a water path between the supply port and the outlet port closed by the open-close valves such that the bactericidal agent is capable of contacting the liquid side surface of the membrane.

19. The gas-dissolved liquid manufacturing apparatus according to any one of claims 11 to 18, wherein the membrane module has a membrane density of 500 - 3000 m²/m³.
